Europäisches Patentamt

European Patent Office

Office européen des brevets

(11) Publication number: **0 288 203 B1**

# EUROPEAN PATENT SPECIFICATION

(45) Date of publication of patent specification: **14.06.95**

(51) Int. Cl.⁶: **C12N 11/04**, C12N 11/08, C12P 1/00

(21) Application number: **88303322.7**

(22) Date of filing: **13.04.88**

(54) **Immobilised cells.**

(30) Priority: **22.04.87 GB 8709470**

(43) Date of publication of application:
**26.10.88 Bulletin 88/43**

(45) Publication of the grant of the patent:
**14.06.95 Bulletin 95/24**

(84) Designated Contracting States:
**AT BE CH DE ES FR GB GR IT LI LU NL SE**

(56) References cited:
EP-A- 0 118 979
FR-A- 2 556 007
US-A- 3 935 069
US-A- 3 989 596

CHEMICAL ABSTRACTS, vol. 106, no. 7, 16th February 1987, page 507, abstract no. 48620m, Columbus, Ohio, US; R.M. ALIEVA et al.

(73) Proprietor: **ZENECA LIMITED**
**15 Stanhope Gate**
**London W1Y 6LN (GB)**

(72) Inventor: **Cantwell, John Burnett**
**35 Sunneymede Drive**
Lydiate
**Merseyside L31 2NR (GB)**
Inventor: **Mills, Paul David Alan**
**29 Orston Crescent**
**Spital**
**Bebington**
**Wirral**
**Merseyside L63 9NZ (GB)**
Inventor: **Jones, Eileen**
**5 ST. Mark"s Crescent**
**Great Sutton**
**Wirral**
**Merseyside (GB)**
Inventor: **Stewart, Ronald Fraser**
**32 Craigstewart Crescent**
**Doonbank**
**AYR KA7 4DB (GB)**

(74) Representative: **Locke, Timothy John et al**
**Intellectual Property Group**
**Zeneca Bioproducts**
**P.O. Box 2**
**Belasis Avenue**
**Billingham**
**Cleveland TS23 1YN (GB)**

## Description

This invention relates to immobilised cells, in particular those in which bacterial or fungal cells are immobilised in intimate admixture with a solid organic polymer, and to processes for the preparation and use thereof.

It is known, for certain biological cells which contain a commercially-useful enzyme, that it is often desirable for the enzyme to be harvested in situ in the cell. Where it is desired to use the aforesaid enzyme in chemical or biochemical processes it is known that it is often convenient to immobilize the cell (including the enzyme) in a strong, porous structure. Such known porous structures, which may be continuous or in the form of a plurality of discrete granules, comprise at least one cell and a material which provides the structural support therefor; which material is hereinafter referred to for convenience as "the support." Such known structures tend to (a) facilitate handling of the cell and/or enzyme; (b) increase the lifetime of the enzyme by allowing its catalytic activity to be maintained and its loss from the cell to be reduced such that the cells can be recycled and re-used; and (c) allow the enzyme to be used in harsh, physical and/or chemical environments.

It has been reported that cells of certain microorganisms have been immobilised in certain systems by inter alia:

(a) adhesion to a solid inorganic surface, e.g., glass (Ballotini) beads;

(b) entrapment within certain plastic sponges or wire meshes;

(c) capture in gels formed from certain suitable materials, e.g. an alginate or a polyacrylamide;

(d) combination with ion-exchange compounds, e.g. Daugulis et al used a basic ion exchange XE352 for the absorption of Saccharomyces cerevisiae;

(e) covalent bond formation to certain solid supports;

(f) entrapment of certain cells, in combinations of polycatonic and polyanionic flocculating agents as described in US 3821086 and by S J Bungard et al in the American Chemical Society Symposium Series 1979, 106, 139;

(g) entrapment of certain cells in certain polymer beads by incorporation of the cells in a monomer mixture prior to the polymerisation thereof;and

(h) cross-linking with glutaraldehyde, often in the presence of a protein, e.g. albumin.

It is known that great care often has to be taken in the preparation of the aforesaid systems to maintain the integrity and accessibility of the cells therein. Furthermore, those procedures which involve chemical formation of the support in the presence of the cells or chemical treatment of cells e.g. with glutaraldehyde, are often subjected to certain constraints, e.g. enzyme degradation.

It will be appreciated that for use in industrial processes it is desirable to combine (a) strong adhesion or bonding of the cells to the support with (b) a high concentration of accessible enzyme in the structure. The strong adhesion or bonding of the cells to the support is such that they are not detached or unduly damaged by the stresses and strains to which they may be subjected in use, for example in continuous reaction columns or batch-process vessels. Further, it is desirable that the structures are able (a) to withstand sustained pressure, e.g. in continuous-reaction columns, without undue compaction thereof and (b) to survive sustained shear forces in batch stirred reactor environments.

We have now found surprisingly that compositions produced from one or more species of cell and one or more polymer latices in particulate form exhibit the aforesaid desirable features and tend not to have many of the aforesaid disadvantages. For example, the cell wall may be stabilised, i.e. so-called cell-wall integrity is retained; deactivation of the useful enzyme within the cell by a cross-linking agent does not occur; the composite is often tough when wet; the ease of processing thereof may be increased; and the cost of such compositions tends to be lower than those of prior art compositions since latices tend to be cheaper than certain prior art polymeric components, e.g. prior art flocculants.

We have found that high molecular weight latex polymer particles tend to have suitable physical properties of a film such as hardness, wear-resistance and other desirable film integrity properties (a) to sufficiently protect discrete cells and (b) to provide sufficient resilience and/or strength to hold the originally discrete cells in an integral structure.

According to the present invention there is provided a composition derived from an intimate mixture of an aqueous latex of a film forming or coating polymer and cells of an organism. The polymer preferably comprises at least one soft polymer.

The present invention also comprises a process for the production of a product of an enzyme reaction, for example an intermediate or a metabolite, which process comprises the step of contacting, under appropriate reaction conditions, a substrate with a composition as defined in the first aspect of the present invention.

According to another aspect of the present invention there is provided a shaped composition derived from an intimate mixture of (a) an aqueous latex of a film-forming or coating polymer and (b) cells of an organism.

A composition according to the invention may be formed by a process which comprises at least the steps of (A) forming an aqueous latex of at least one film-making soft polymer;

(B) admixing said aqueous latex with cells of an organism; and

(C) allowing or causing the admixture to form agglomerates.

A composition is according to the present invention may be prepared by a process which comprises at least the steps of (A) mixing cells of the organism with a latex of at least one soft polymer such that an intimate mixture if fomred; (B) allowing or causing the intimate mixture to form agglomerates, without loss of this intimacy, by any means known or to be discovered; and (C) recovering the agglomerates wherein in at least one of the steps A, B or C the soft polymer particles "flow-out" or coat at least a portion of the cells.

By "soft polymer" we mean that under the conditionsused in the process according to the second or fifth aspects of the present invention;

(a) the polymer has sufficient fluidity to provide flow-out or undergo at least partial coalescence eg by, it is believed, the capability of segments of the polymer chains to move relative to each other, such that at least a portion of the cells of the organism are coated therewith; and

(b) the aforesaid sufficient fluidity is obtained at a temperature which is lower than the highest temperature which the particular enzyme within the particular integral immobilised cell can withstand.

It will have sufficient resilience or strength such that it can hold the originally discrete cells in an integral structure.

Preferably the aforesaid sufficient fluidity is obtained by carrying out at least one step of the process of the present invention at a temperature which is above the Tg of the soft polymer. However, we do not exclude the possibility that an additive, eg a plasticiser therefor, may be present in the admixture such that the sufficient fluidity may be obtained. Furthermore, the fluidity may be increased by use of the additive, eg by further softening the soft polymer, such that a thinner skin thereof is provided on the cells.

By "agglomerates" we mean multi-particulate or granular species formed by the mutual adhesion of many previously discrete and independently moving particles. Such mutual adhesion is a typical property of colloidal systems and is variously referred to in the art by the terms, flocculation, coagulation, and aggregation; the larger species produced being known as flocculates (or flocs), coagulates, and aggregates. These terms are sometimes defined and distinguished in the light of the procedure used to bring about the mutual adhesion and the forces holding the originally discrete particles together in the agglomerate. However, there is a tendency in the art to use them indiscriminately and, accordingly, where we use "agglomerates" and "agglomeration" it is to be understood that they include "flocculates", "coagulates" and "aggregates"; and "flocculation", etc. As examples of known processes by which agglomeration of a colloidal system may be effected may be mentioned inter alia (a) the addition of an electrolyte, (b) the addition of a soluble polymer, (c) pH adjustment, (d) mechanical treatment such as rapid stirring, shearing, or centrifugation, and (e) contacting with a liquid which is immiscible with the liquid phase of the colloid. It will be appreciated that the means used for the preparation of a particular agglomerate will be chosen in the light of inter alia the type of forces opposing mutual adhesion of the discrete particles. Where at least two kinds of discrete particles are present the agglomeration step is more correctly referred to as co-agglomeration (or, corresponding, co-flocculation, co-coagulation, or co-aggregation). Moreover where, as in the compositions of the first aspect of this invention, the resulting agglomerates contain, in intimate admixture, a plurality of types of particles which were initially discrete (i.e. the agglomeration process is not accompanied by partial or complete segregation of the several types), the agglomerates are more correctly referred to as hetero-agglomerates (or correspondingly, hetero-flocculates, hetero-coagulates, or hetero-aggregates).

As examples of organisms of which compositions according to the first aspect of the present invention may be comprised may be mentioned inter alia plant cells, animal cells, or preferably cells of microorganisms, for example fungi, algae or preferably bacterial cells, or more preferably cells of a Gram-negative bacterium. As specific examples of such bacterial cells may be mentioned inter alia Pseudomonas sp, e.g. Pseudomonas putida; Arthobacter sp; Bacillus sp eg Bacillus subtilis; or Enterobacteriacae sp, e.g. E.coli.

Whereas it is often preferred that the aspect ratio of the cells present in the composition according to the present invention is low, eg less than 10, typically about 3 or 4, we do not exclude the possibility that they may have higher aspect ratios. For example, they may be filamentous microorganisms, e.g. fungi. As examples of fungi may be mentioned inter alia strains of Fusarium, Rhizobium Oligosporus, Rhizopus and Aspergillus, e.g. Fusarium graminearum and lateritium. It will be appreciated that such filamentous microorganisms may have short side-branches attached to the mains stem of hypha.

3

Whilst it is often preferred that discrete cells are employed we do not exclude the possibility that microorganisms which are morphologically distinct from the aforesaid cells may be used; a mass of attached or entangled cells, e.g. filamentous fungi, may be used.

As examples of desirable enzymes which are present in the aforesaid cells may be mentioned inter alia glucose isomerase, dehalogenase, aromatic oxygenase, cyanide hydratase and catalase.

We do not exclude the possibility that a mixture of two or more strains or species of cells may be present, particularly where the presence of a plurality of compatible enzymes is desired, e.g. where a first enzyme acts at one stage of a process and a second enzyme acts at a later stage of the process according to the third aspect of the present invention.

Preferably discrete cells, where they are used in the process according to the second or fifth aspects of the present invention, are unprocessed but we do not exclude the possibility that stored or processed mature cells, e.g. reconstituted freeze-or other suitable-dried suspensions thereof, may be used.

The organism of which the composition according to the first aspect of the present invention is comprised may comprise genetically modified cells. By "genetically modified cell" we mean that the cell contains a gene (which may be natural or synthetic) which the cell does not contain in its natural environment, As examples of typical host organisms which may be genetically modified may be mentioned inter alia E.coli, Methylophilus methylotrophus, B.subtilis and Saccharomyces cerevisiae.

By "latex" we mean a dispersion or suspension of small, e.g. less than 10 microns, polymeric particles in a liquid. The dispersed particles may be made by processes known in the art, e.g. comminution, or preferably by suspension, dispersion or emulsion polymerisation techniques. The aforesaid liquid is chosen such that it does not adversely affect the cells with which it is to be intimately mixed in the process according to the second or fifth aspects of the present invention, preferably it is polar hydroxy and more preferably is aqueous. The aforesaid polymeric particles may have any shape, they are often spherical, but we do not exclude the possibility that they may have an alternative shape, e.g. ellipsoid.

Aqueous based coating compositions based on aqueous latices of film-forming polymers are well known, eg the polymer latices are typically prepared by aqueous emulsion polymerisation of one or more monoethylenically unsaturated monomers. Such a preparation is typically carried out in an aqueous medium with the aid of a free-radical-yielding initiator and usually in the presence of a surface active agent, which will yield an aqueous latex. The latex may also be formed by inter alia post-emulsification (in water) of pre-formed solid polymers prepared by any suitable addition polymerisation technique, eg free radical, anionic or cationic polymerisation.

The use of a soft polymer in the process according to the second or fifth aspects of the present invention tends to prevent deactivation of the protein or enzyme, where mature cells are used, or where certain desirable characteristics of the cells and/or enzyme, e.g. cell viability, cannot withstand a temperature above a certain temperature. For example, where the cells or the enzyme therein can withstand a temperature of up to about 60°C, e.g. Arthrobacter, the polymer may have a Tg up to about 60°C; where the cells can withstand a temperature of up to about 30°C, e.g. Pseudomonas putida, the polymer may have a Tg up to about 30°C. As examples of commercially available suitable latices for use in the process according to the second or fifth aspects of the present invention may be mentioned inter alia copolymers of methyl methacrylate and copolymers of styrene with, e.g. butyl acrylate, ethyl acrylate and ethyl hexyl acrylate, the Tg's of which copolymers often are in the range from 0°C up to the Tg of the homopolymer; polyvinyl acetate; polyvinylidene chloride and suitable copolymers thereof, e.g. having a Tg in the temperature range from -5°C to 15°C; butadiene-styrene copolymers, i.e. so-called "SBR rubbers", e.g. having a Tg of less than -10°C, e.g. about -60°C. The skilled man will readily find by experiment a suitable low Tg polymer suitable for use with a particular cell in the process according to the second or fifth aspects of the present invention.

It will be appreciated that the structure and permeability to aqueous media of compositions according to the present invention is such that a substrate is allowed access to the cells containing the enzyme to which it is to be subjected; a composition allowing suitable water permeability is used, e.g. acrylates are often preferred to polyvinylidene chloride (which tends to be a barrier to $H_2O$).

It will be appreciated that the latex which is used in the process according to the second and fifth aspects of the present invention is preferably colloidally stable at the pH at which it is mixed with the cells, at least for the time span of mixing.

The at least one soft polymer latex is chosen such that a desirable balance of properties, e.g. mechanical strength and compressive strength, is obtained in the composition. We have found that by using a mixture of latices of suitable hard and soft polymers in the process according to the second or fifth aspects of the present invention, a structure having a suitable balance of properties may be obtained; the aforesaid hard latex tends to be co-flocculated into the mass but does not flow significantly under the conditions at which the process according to the present invention is carried out. The skilled man, where he wishes to use a mixture of hard and soft polymer in the composition according to the present invention, will be readily able to find such a mixture by experimentation.

The film-forming soft polymer is preferably a polymer or copolymer comprising units derived from one or more monoethylenically unsaturated monomers of the film-forming type containing one or more olefinic, vinyl, vinylidene groups or preferably an acrylic or methacrylic double bond.

In the process according to the second aspect of the present invention, the pH of the latex is checked for compatibility with the cells and, where necessary, is adjusted accordingly such that it is compatible with the cells and the desired enzyme contained therein. The latex is mixed with a suspension of the cells, preferably the mixture is stable such that flocculation on contact does not occur and an even distribution of the cells in the latex is obtained. However, we do not exclude the possibility that, where an uneven distribution of cells in the composition is desirable, an uneven distribution of cells in the latex is used.

The admixture of the latex with the cells may be effected in any suitable manner or order. For example, the cells may be stirred into the preformed polymer latex, allowing sufficient time for the cells to be evenly distributed throughout the latex; this leads to a more homogeneous composition with better mechanical stability properties.

A suitable flocculating agent is then often added. However, where there is an appropriate interaction between the polymer particles and the cells, e.g. the surface of the cells is negatively charged and the surface of the polymer particles is positively charged, addition of a flocculating agent may be unnecessary; the absence of a flocculating agent often confers no technical advantage on the process or the product thereof.

Where co-flocculation of the aforesaid mixture of cells and polymer particles is effected by addition of a suitable flocculant, the suitable flocculant may be an inorganic or an organic substance. As examples of known ionic flocculants, which may be simple or complex, may be mentioned inter alia those derived from cations such as $Na+$, $Ca2+$, $Mg2+$, $Ba2+$, $Fe^{3+}$, $Al3+$ or uranium oxide cations. As examples of known organic polymeric flocculants, which may be cationic, anionic or nonionic, may be mentioned inter alia poly-(ethylene imine), poly(acrylic acid) and poly(acrylamide). The flocculants are chosen such that inter alia there is minimal loss in activity of the desired enzymes present in the cells. At the flocculating stage, it is often possible to control the structure within the flocculated mass, for example to produce domains of cells, e.g. a plurality of contiguous cells, using known procedures, similar to those reported by Jones, Mills, Stewart and Sutton at the Conference on Ceramic Science and Technology, Boston, USA, August 3-6, 1986.

The flocculating agent is preferably directly effective on the latex; where one component of a system comprising a two component mixture becomes unstable the system is often flocculated. For example, Arthrobacter is a very colloidally stable cell which does not usually flocculate with calcium chloride, however, where Arthrobacter is mixed with an appropriate latex, the latex is destabilised by the calcium chloride and the system flocculates.

The flocculated composition recovered from step C of the process according to the second aspect of the present invention tends to be self-supporting, e.g. in the form of a cheese or typically a putty, albeit having a wide size distribution of aggregates, and not paste-like or crumbly. The flocculated composition often remains intact when filtered and may be used without further drying for the reaction thereof with the desired substrate but this is not preferred.

Where the flocculation step is carried out at a temperature above the Tg of the latex used in the process according to the second or fifth aspects of the present invention there is a tendency for so-called coalescence of the latex to occur. Such samples may be further consolidated, i.e. to reduce water content thereof, by filtration or centrifugation and the consolidated composition produced may be further shaped e.g. be extruded into fibrils. These fibrils may be used without further drying but this is not preferred.

Typically, the composition is dewatered by filtration or centrifugation and the consolidated mass can then be extruded such that fibrils are produced. However, we do not exclude the possibility that alternative means of shaping the composition such that a strong, porous structure which allows ready access of the substrate to the cells may be employed. For example, the intimate mixture of cells and latex may be aggregated and shaped into granular form by known agglomerative or alternative techniques, e.g. spray drying or spheronisation.

Where fibrils are prepared they may be dried at a temperature which will allow the latex particles to flow but does not deactivate the desired enzyme. The dried fibrils may be cut up and stored ready for use, or they may be used undried.

It is sometimes preferred that a first liquid (in addition to the liquid medium of which the latex is comprised) is employed in the process according to the second aspect of the present invention, which liquid is compatible with the cells and is capable of collecting the co-flocculated latex and cells, i.e. by so-called "spherical agglomeration techniques", which include liquid bridging as is more fully described by C.E. Capes in "Handbook of Powder Technology, Volume 1, Particle Size Enlargemnent", Elsevier, 1980. By "colllecting" we mean that there is a high per-centage of cells with latex incorporated into the first liquid such that granules are formed which may then be used as hereinbefore described. As an example of a suitable liquid may be mentioned inter alia heptane.

As an example of an additive which may be added to further soften the soft polymer may be mentioned methylene chloride (for vinylidene chloride/polyvinyl chloride copolymers).

The unusually high colloidal stability of certain cells, e.g. Arthrobacter, to certain variations in the liquid with which it is in contact, e.g. inorganic salt concentrations, tends to complicate certain potential harvesting routes thereof. By the process of the present invention the aforesaid complications can often be overcome such that the cells (i) can be harvested from suspension and be shaped, e.g. into granules/fibrils, for packing into a column, (ii) retain their enzyme activity, (iii) be sufficiently porous to allow liquid access to the cells through the matrix, (iv) have sufficient strength to withstand the pressure inside the aforesaid column (v) and be substantially resistant to break-up and wash off.

The mutual adhesion forces promoted by the aforementioned flocculating agent typically confer upon agglomerates and hetero-agglomerates sufficient stability such that they retain their integrity during subsequent steps of their preparation such as gentle stirring or concentration by, for example, filtration or centrifuging. However, the pressures and shear forces to which they may be subjected in reactors and process vessels tend to cause disintegration, or significant erosion thereof, in times much shorter than their desired, and otherwise achievable, bio- catalytic life-times. It is therefore a preferred feature of the second and fifth aspects of this invention that, for at least a part of the time during or subsequent to the agglomeration step the temperature of the composition is above the Tg of the soft polymer. This allows some fusion of contiguous polymer particles and hence consolidation and strengthening of the agglomerate structure. It will be appreciated, as hereinbefore described, that the Tg of the polymer should not exceed the highest temperature which the enzyme within the cell or the cell integrity can withstand. There is, however, no such clearly defined lower limit to Tg since, being stabilised against mutual adhesion, the polymer particles often will not fuse and consolidate prior to agglomeration. It is believed, without prejudice to the present invention, that in the composition of the first aspect of the present invention, the polymer particles have, to a greater or lesser extent, lost their identity and have become fused into porous structure, which may be in the form of discrete granules or a continuous matrix or a mixture thereof.

The composition of the invention may also include certain further ingredients. For example, it may include ingredients often employed in film-forming coating formulations, such as defoamers, rheology control agents, thickeners, dispersing and stabilising agents, wetting agents, extenders, coalescing solvent, plasticisers etc.

The present invention is further illustrated with reference to the following Examples.


Example 1


This Example illustrates (a) the activity of an enzyme in and (b) the mechanical stability, and the ease of recovery, of certain of the immobilised cells according to the present invention.

A strain of Pseudomonas putida containing the enzyme D-2-monochloropropionic acid dehalogenase was cultured. A dispersion of the cells, containing 10% w/w dry basis of solid matter, was added to a mixture of an acrylic copolymer latex (mean diameter 0.4 microns; Tg = 5°C) at 10% w/w solids and Styrene-Butadiene rubber (SBR) latex (mean diameter 0.1 microns; Tg less than -10°C) at 10% w/w solids such that the proportion of cells to acrylic latex to SBR latex was 2:1:1 on a dry weight basis. The dispersion was then mixed and 10% poly(ethylene imine) (PEI) solution (MW 50,000 -100,000) was added with rapid stirring to induce aggregation in the mixture. The mixture was then gently stirred at between 50 to 100 RPM.

The resultant aggregates were separated from the supernatant by centrifugation. From the consolidated mass, fibrils of approximately 1-2 mm in diameter were produced by extrusion. These were dried overnight at room temperature.

The activity of the dehalogenase enzyme in the immobilised system in a neutralised and buffered 2-chloropropionic acid substrate was measured in a bioreactor containing 10g/litre of immobilised cells (dry basis) and stirred at 100 RPM. The initial activity was 1.4 milli-Moles (mM) of chloride per hour per gram dry weight of cells. The reaction went to stoichiometric completion. After reaction the supernatant turbidity was assessed at 575 nm using a visible spectrophotometer (Pye Unican SP6-300) and disposable cuvette (1cm pathlength). The optical density was found to be 1.28 indicating a relatively low degree of attrition of the fibrils (compare First Comparative Test).

6

In a first Comparative Test, the above procedure was repeated except that "free" i.e. non-immobilised, cells were used instead of cells immobilised in a mixture of acrylic copolymer latex and SBR latex. The optical density of the supernatant was found to be more than 2.0.

The occurrence of cell lysis was determined by monitoring the soluble protein concentration in the supernatant following the reaction. The determination was carried out using the Bio-Rad proteins-assay procedure. The soluble protein concentration for the latex-immobilised system was much lower at 0.77 mg/ml than that produced in the first Comparative Test using "free" cells, 1.2 mg/ml.

The latex-immobilised cells were easily isolated from the reaction liquor by decantation. In contrast, the "free" cell treated system was difficult to isolate requiring use of flocculating agents and centrifugation.

## Example 2

This Example illustrates the repeated use of immobilised cells according to the present invention.

The procedure described in Example 1 was repeated to give latex-immobilised cells containing 50% by weight dry cells which were used to carry out the biotransformation described in Example 1.

The immobilised cells were found to have an initial activity of 1.2mM of $Cl^-$/hour/gram and the reaction went to completion. The biocatalyst was isolated by decantation and added to a fresh batch of substrate in the bioreactor. This second biotransformation also went to completion but with a reduced initial rate (0.25mM of $Cl^-$/hour/gram). The procedure was further repeated and again the biotransformation occurred.

In a Comparative Test using "free" cells, after re-isolation by centrifugation, they yielded no measurable catalytic action for the second and subsequent attempts at biotransformation.

Hence, the latex-immobilisation confers upon the cells a stabilising influence for the dehalogenase enzyme compared with "free" cells. This stabilising influence permits re-use of the latex-immobilised catalyst whereas there is a tendency for no re-use for the "free" cells.

## Example 3-7

These Examples illustrate the use of a range of flocculants and the use of a hard polymer as well as the soft polymer.

In these Examples, the procedure of Example 1 was repeated except that the compositions shown in Table 1 were used. The cells and each polymer were used at a concentration of 10% w/v. In the Comparative Test two portions of 10% w/v poly(methyl methacrylate) were used.

7

TABLE 1

| Example No. | 1st Soft Polymer (S1) | 2nd Soft Polymer (S2) | Hard Polymer (H) | Floccul- ant or coagul- ant | Ratio Dry Wt C:S1:S2:H |
|---|---|---|---|---|---|
| 3 | – | SBR | PMMA(1) | PEI | 4:0:3:3 |
| 4 | Acrylic | SBR | – | Zeetag 63 | 4:3:3:0 |
| 5 | Acrylic | SBR | – | $CaCl_2$ | 4:3:3:0 |
| 6 | Acrylic | SBR | – | $Al(NO_3)_3$ | 4:3:3:0 |
| 7 | Acrylic | SBR | – | $FeCl_3$ | 4:3:3:0 |
| CT | – | – | PMMA(2) | | 2:0:0:3 |

CT is a Comparative Test

C: <u>Pseudomonas putida</u>

PMMA(1 and 2):Poly(methyl methacrylate)-based latices of particle size 0.3 microns and having Tg's of (1) above 90°C and (2) 87°C respectively.

PEI: Poly(ethylene-imine) of Mw 50,000-100,000 (ex Polysciences).

Zeetag: ex Allied Colloids.

In Examples 3-7, resilient immobilised cell-containing fibrils were obtained which survived a 24 hour exposure to agitation (100 RPM) in buffered 0.5M CPA substrate. At the end of the reaction, the fibrils were readily separated from the reaction mixture by decantation, a substantially clear supernatant was left.

In the Comparative Test, extensive attrition of the fibrils occurred thus causing generation of a very turbid supernatant which had an optical density of more than 2.0.

Example 8

This Example illustrates particles of higher aspect ratio and having higher initial activity prepared from immobilised cells according to the present invention.

The procedure of Example 1 was repeated except that a die (of smaller diameter) was used, fibrils of 0.8mm diameter were obtained.

At the end of the biotransformation reaction, which went to completion, these fibrils were mechanically intact. However, the initial activity of these fibrils was found to be 66% greater than that of the 1-2mm diameter fibrils used in Example 1.

Example 9

This Example illustrates the resilience to high substrate concentration of certain immobilised cells according to the present invention.

A batch of fibrils prepared as in Example 1 was exposed in a bioreactor to a neutralised buffered substrate at a concentration of 2.5 M chloro-propionic acid. The immobilised system continued to catalyse the reaction over a prolonged period of more than 48 hours.

In Comparative Tests with "free" cells at a range of substrate concentration of chloro-propionic acid, the half-lives of the enzyme were found to be as shown in Table 2.

## TABLE 2

| Example No. | Concentration CPA (M) | Half-life (hours) |
|---|---|---|
| CT1 | 0.55 | 20 |
| CT2 | 0.65 | 10 |
| CT3 | 0.85 | 7 |
| CT4 | 1.15 | 2.5 |
| CT5 | 1.4 | 1.5 |
| CT: Comparative Test | | |

From these results it can be extrapolated that at a substrate concentration of 2.5 M chloropropionic acid, substantially total inactivation of the enzyme in "free" cells would occur in about 2 hours. Furthermore, in these Comparative Tests, we found that the reaction reached less than 10% of the theoretical conversion.

Example 10

This Example illustrates immobilised cells comprising a different microorganism.

The procedure for the preparation of fibrils described in Example 1 was repeated except that Bacillus subtilis was used instead of Pseudomonas putida. The mechanical stability and the catalytic strength of these fibrils were determined. The catalytic strength was determined by their ability to catalyse, via the naturally occurring enzyme catalase, the decomposition of a hydrogen peroxide substrate.

Mechanical integrity was monitored by agitating the fibrils in 1 Molar phosphate buffer (pH 7.2) at 100 RPM, 35°C for a period of about 24 hours. The supernatant optical density was then recorded at 575 nm as hereinbefore described. A very low supernatant optical density was measured (0.021) corresponding to an essentially clear supernatant with mechanically intact biocatalyst fibrils.

The activity of this system was assessed by volumetric determination of the oxygen product evolved following the introduction of a portion (25 mls) of a 0.02M phosphate buffer (pH7) containing 9.5M hydrogen peroxide solution (0.7 mls) into an agitated flask containing a sample (0.2g) of the fibrils.

The reaction went to stoichiometric completion in approximately 30 minutes at 23°C. The fibrils were isolated by decantation and high residual activity was demonstrated on re-use. The reaction again proceeded to stoichiometric completion.

Example 11

This Example illustrates the use of a yet further microorganism in immobilised cells according to the present invention.

A composition was prepared and tested as in Example 10 but substituting cells of Escherichia coli for Bacillus subtilis. Good mechanical integrity was demonstrated by agitating the fibrils in 0.1M phosphate buffer at approximately 20°C at 100 RPM for 24 hours. The optical density of the supernatant (recorded at 575 nm) was less than 0.07. The fibrils were mechanically intact at the end of the 24 hours. Broad initial activity and re-use capability of the biocatalyst were found using the catalase assay described in Example 10.

Example 12

This Example illustrates the use of a filamentous fungus (i.e. a microorganism of high aspect ratio which has a very different morphology from the microorganisms used in Examples 1-11). The fungus used, namely Fusarium lateritium, contained the enzyme cyanide hydratase.

A suspension (25 grams) containing approximately 5% w/w (dry basis) of the filamentous organism Fusarium lateritium (in viable form) was mixed with gentle stirring with a mixture (25 grams) of a 5% w/w mixed dispersion, of the "soft" acrylic latex and SBR latex (equal concentration, on weight basis) described in Example 1. A 10% solution of PEI was then added with more vigorous stirring (600 RPM) such that the final concentration of flocculant was about $2\frac{1}{2}$% w/w on total solids. Aggregates were readily isolated by filtration, shaped into fibrils by extrusion and then allowed to dry at ambient temperature.

Examples 13-17

These Examples illustrate the mechanical robustness of further compositions according to the present invention.

The compositions used in Examples 13-17 were prepared from the same microorganism under the conditions described for Example 12, except that the ratio of fungi to latex are as shown in Table 3. Fibrillar biocatalysts were obtained, the mechanical robustness of which was assessed by prolonged shaking at 100 RPM in 0.1M Trizma buffer (Sigma, pH 8.5) following the addition of 0.5 ml of 0.2 M sodium azide to prevent extraneous bacterial growth. The concentration of fibrils was such that the agitated suspensions contained 0.1g of fungal material per $20cm^3$ of buffer. After 24 hours the supernatant was substantially clear with very little attrition of the fibrils. The optical density (at 575 nm) of the supernatant after 24 hours agitation was measured; the results are shown in Table 3.

TABLE 3

| Example No. | Wt Ratio Fungus:Latex | OD 575 reaction liquor |
|---|---|---|
| 13 | 90 : 10 | 0.011 |
| 14 | 70 : 30 | 0.014 |
| 15 | 50 : 50 | 0.011 |
| 16 | 30 : 70 | 0.010 |
| 17 | 10 : 90 | 0.018 |

An assay for cyanide hydratase activity confirmed that the enzyme activity was substantially preserved during the preparation of the immobilised system.

Example 18

This Example illustrates the use of a yet further microorganism in immobilised cells according to the present invention.

Arthrobacter culture (2.5 litres; containing 10g/litre of viable cells) was mixed with a 10% w/w latex (78 mls) of a poly(vinylidene chloride/vinyl chloride/ 2-ethyl-hexyl acrylate) (Tg 10-13°C). Molar CaCl$_2$ (156 mls) was added with slow stirring (50-100 RPM) to the mixture of cells and latex which was then flocculated. The flocculated mass was separated from the supernatant by centrifugation. The consolidated mass was extruded onto a non-stick surface via a syringe, fibrils of approximately 2 mm diameter were obtained. These were dried at 60°C overnight and the dried fibrils were found to contain 76% Arthrobacter cells be weight.

The strength of the fibrils, determined by so-called Nz analysis, was 765 Nz x 10$^{-5}$ Kg$^2$ m$^{-2}$s$^{-2}$; they had an activity of 70 glucose isomerase units per gram. Nz analysis gives a numerical measure of the resistance of a bed of material to time-dependent irreversible creep or deformation which is induced by fluid friction. It provides an indication of the capability of a material to function in a mechanically stable manner under conditions of fluid flow through a bed thereof.

To carry out the analysis, the pressure drop across a swollen bed of material of known weight is measured at a series of known liquid flow rates and top loadings. From these measurements, the bed permeabilities (Z), at a range of stress pressures (Ps), can be calculated from the equations:

$$Z = \frac{FuW}{A^2 pg(h-hc)} \quad kg/m$$

$$Ps = \frac{Mg}{A} + (h - hc)\, pg \quad N/m^2$$

where (at a defined temperature):

W = Weight of dry material for forming the bed (kg);

A = Cross-sectional area of the column ($m^2$);

F = Solution flow rate ($m^3/s$);

u = Solution viscosity ($Ns/m^2$)

p = Solution density ($kg/m^3$);

h = Solution head loss across the bed (m);

hc = Solution head loss across the apparatus at flow rate F (m);

g = Gravitational constant ($m/s^2$)

M = Applied top loading (kg);

A graphical integration of Z with respect to Ps is carried out between two preset limits (eg $5 \times 10^{-7}$ kg/m for Z and 20 $KN/m^2$ for Ps). Nz is represented by the area under this curve, ie

$$Nz = \int^{20.10^3} Z\, dPs \quad kg^2/m^2/s^2$$

with the proviso that Z is less than $5 \times 10^{-7}$ kg/m.

## Example 19

This Example illustrates the use of a liquid to produce particles having different morphology.

Arthrobacter culture (2.5 litres at 10g/l dry weight) was co-flocculated with a poly(vinylidene chloride/vinyl chloride/2-ethyl-hexyl acrylate) latex (390 mls at 10% by weight) using $CaCl_2$ as described in Example 13. Prior to the centrifugation, methylene chloride (100 mls) was added with stirring.

This solvent served both to "collect" the flocs prior to centrifugation and also to soften the latex via plasticisation. In this Example, fibrils were formed by extrusion but drying was carried out at a temperature, i.e. 25°C, which was lower than that used in Example 18. The fibrils were robust and showed no break-up when shaken in a glucose syrup (45% glucose, w/v, at 60°C, pH 8.5) at 200 RPM overnight. Satisfactory activity was demonstrated by isomerisation of D-glucose to D-fructose at 15 glucose isomerase units per gram of biocatalyst. Nz analysis showed a fibril strength of more than $1039Nz \times 10^{-5}$ $kg^2$ $m^{-2}S^{-2}$.

## Example 20

This Example illustrates the use of a plasticising agent and liquid to produce particles having a different morphology.

A composition containing 13% w/w (dry basis) of Arthrobacter cells and 87% (0.1μm) poly(vinyl chloride) latex was prepared using 0.1M $BaCl_2$ as the flocculating agent. The flocs were then collected by the spherical agglomeration technique using a solution of dioctyl phthalate in hexane as the bridging liquid. Strong granules were obtained; they were sufficiently resilient to survive the shaking test (described in Example 19) in glucose syrup with substantially no granule destruction. They were found to have an activity of 10 glucose isomerase units per gram of granule.

In a comparative test, neat hexane was used instead of the solution od dioctyl phthalate in hexane as the bridging liquid. The granules obtained were mechanically weak when tested in the aforesaid shaking test.

## Claims

1. A composition derived from an intimate mixture of an aqueous latex of a film forming or coating polymer and cells of an organism.

2. A composition as claimed in Claim 1 in the form of a paste or putty.

3. A composition as claimed in Claim 1 in the form of a shaped mass such as fibres or granules.

4. A composition as claimed in Claim 1 wherein the Tg of the polymer is below the highest temperature which the enzyme in the cell can withstand without becoming unduly adversely affected thereby.

5. A composition as claimed in any preceding Claim wherein the cells comprise bacterial cells.

6. A composition as claimed in any preceding claim wherein the bacterial cells comprise Pseudomonas sp, Arthrobacter sp, Bacillus sp, or Enterobacteriacae sp.

7. A composition as claimed in Claim 1 wherein the cells comprise at least one of the following enzymes: glucose isomerase, dehalogenase, catalase, oxygenase or cyanide hydratase.

8. A composition as claimed in any preceding Claim wherein the polymer is a copolymer of methyl methacrylate, a copolymer of styrene, poly (vinylidene chloride) and suitable copolymers therefore or butadiene-styrene copolymers.

9. A composition as claimed in any preceding Claim further characterised in that the particulate solid comprises both a hard and a soft polymer.

10. A process for the preparation of a composition for providing an enzyme used in a chemical or biochemical process which composition comprises an agglomerate of cells of an organism comprising the enzyme intimately dispersed in a structure which is, or is derived from, a plurality of particles of an organic solid: characterised in that (i) the particulate solid comprises at least one soft polymer which has sufficient fluidity to provide flow-out or undergoes at least partial coalescence such that at least a portion of the cells of the organism are coated therewith, in which the aforesaid sufficient fluidity is obtained at a temperature which is lower than the highest temperature which the enzyme within the particular integral immobilised cell can withstand and (ii) the process comprises at least the steps of:
    (A) mixing cells of the organism with a latex of at least a soft polymer which polymer has sufficient fluidity to provide flow-out or undergoes at least partial coalescence such that at least a portion of the cells of the organisms are coated therewith, such that an intimate mixture is formed;
    (B) allowing or causing the intimate mixture to form agglomerates; and
    (C) recovering the agglomerates.
    wherein in at least one of the steps A, B or C, the polymer particles flow-out or undergo at least partial coalescence or coat at least a portion of the cells.

11. A process as claimed in Claim 11 wherein for at least part of the time during, or subsequent to, Step B, the temperature is maintained above the Tg of the soft polymer.

12. A process for the preparation of a composition as claimed in any one of Claims 1 to 9 which process comprises the steps of:
    (A) forming an aqueous latex of at least one film-forming polymer;
    (B) admixing said aqueous latex with cells of an organism; and
    (C) allowing or causing the admixture to form agglomerates.

13. A process as claimed in Claims 10, 11 or 12 further characterised in that the composition is subjected to a shaping process.

13

EP 0 288 203 B1

**14.** A process according to Claims 10, 11 or 12 wherein at least one step of the process is carried out at a temperature which is above the Tg of the soft polymer.

**15.** A process as claimed in Claim 10 wherein a suitable flocculant is used to aid flocculation.

**16.** A process as claimed in Claim 15 wherein the flocculant is directly effective on the latex.

**17.** A process as claimed in Claim 16 wherein the suitable flocculant is, or is derived from, a cation such as $Na^+$, $Mg^{2+}$, $Ba^{2+}$ and $Al^{3+}$ or a uranium oxide cation.

**18.** A process for the production of a product of an enzyme reaction which process comprises the step of contacting a substrate with a composition as claimed in Claim 1 under conditions such that the enzyme reacts on the substrate.

**19.** A process as claimed in Claim 18 wherein the composition comprises cells as defined in Claim 6.

**Patentansprüche**

**1.** Zusammensetzung, die von einer innigen Mischung aus einem wäßrigen Latex aus einem Filmbildungs- oder Beschichtungspolymer und Zellen von einem Organismus abgeleitet ist.

**2.** Zusammensetzung nach Anspruch 1 in der Form einer Paste oder eines Kitts.

**3.** Zusammensetzung nach Anspruch 1 in der Form einer geformten Masse wie beispielsweise Fasern oder Körnchen.

**4.** Zusammensetzung nach Anspruch 1, bei der die Tg des Polymers unterhalb der höchsten Temperatur ist, welcher das Enzym in der Zelle standhalten kann, ohne, daß es durch sie in ungeeigneter Weise nachteilig beeinflußt wird.

**5.** Zusammensetzung nach einem der vorhergehenden Ansprüche, bei der die Zellen Bakterienzellen umfassen.

**6.** Zusammensetzung nach einem der vorhergehenden Ansprüche, bei der die Bakterienzellen die Arten <u>Pseudomonas</u>, <u>Arthrobacter</u>, <u>Bacillus</u> oder <u>Enterobacteriacae</u> umfaßt.

**7.** Zusammensetzung nach Anspruch 1, bei der die Zellen mindestens eines der folgenden Enzyme umfassen: Glukoseisomerase, Dehalogenase, Katalase, Oxygenase oder Cyanidhydratase.

**8.** Zusammensetzung nach einem der vorhergehenden Ansprüche, bei der das Polymer ein Copolymer aus Methylmethacrylat, ein Copolymer aus Styrol, Poly(vinylindenchlorid) und dessen geeignetes Copolymeres oder Butadien-Styrol-Copolymeres ist.

**9.** Zusammensetzung nach einem der vorhergehenden Ansprüche, weiter dadurch gekennzeichnet, daß der teilchenförmige Feststoff sowohl ein Hart- als auch ein Weichpolymer umfaßt.

**10.** Verfahren zur Herstellung einer Zusammensetzung, um ein Enzym bereitzustellen, welches in einem chemischen oder biochemischen Verfahren verwendet wird, wobei diese Zusammensetzung ein Agglomerat von Zellen von einem Organismus mit dem Enzym umfaßt, welche innig in einer Struktur dispergiert sind, die eine Vielzahl von Teilchen aus einem organischen Feststoff ist oder von ihnen abgeleitet ist, dadurch gekennzeichnet, daß (i) der aus Teilchen bestehende Feststoff mindestens ein Weichpolymer umfaßt, welches eine ausreichende Fließfähigkeit hat, um Ausfließen bereitzustellen oder sich einer mindestens teilweisen Koaleszenz unterzieht, so daß mindestens ein Bereich der Zellen des Organismus mit dem Weichpolymer beschichtet wird, wobei die vorstehende ausreichende Fließfähigkeit bei einer Temperatur erhalten wird, welche niedriger als die höchste Temperatur ist, der das Enzym in der speziellen, integrierten immobilisierten Zelle standhalten kann, und (ii) das Verfahren mindestens die folgenden Schritte umfaßt:

14

(A) Vermischen der Zellen des Organismus mit einem Latex aus mindestens einem Weichpolymer, wobei das Polymer eine ausreichende Fließfähigkeit hat, um Ausfließen bereitzustellen oder sich mindestens einer teilweisen Koaleszenz unterzieht, so daß mindestens ein Bereich der Zellen des Organismus mit dem Polymer beschichtet wird, so daß eine innige Mischung gebildet wird;

(B) Zulassen oder Verursachen, daß die innige Mischung Agglomerate bildet; und

(C) Wiederherstellung der Agglomerate, worin in mindestens einem der Schritte A, B oder C die Polymerteilchen ausfließen oder zumindest teilweise Koaleszenz zeigen oder mindestens einen Teil der Zellen beschichten.

11. Verfahren nach Anspruch 10, bei dem die Temperatur oberhalb von Tg des Weichpolymers mindestens zu einem Teil der Zeit während Schritt B oder auf Schritt B folgend gehalten wird.

12. Verfahren zur Herstellung einer Zusammensetzung nach einem der Ansprüche 1 bis 9, wobei das Verfahren die folgenden Schritte umfaßt:

(A) Bilden eines wäßrigen Latex aus mindestens einem Filmbildungspolymer;

(B) Vermischen des wäßrigen Latex mit Zellen eines Organismus; und

(C) Zulassen oder Verursachen, daß die Mischung Agglomerate bildet.

13. Verfahren nach einem der Ansprüche 10, 11 oder 12, ferner dadurch gekennzeichnet, daß die Zusammensetzung einem Formungsprozeß unterzogen wird.

14. Verfahren nach einem der Ansprüche 10, 11 oder 12, bei dem mindestens einer der Schritte des Verfahrens bei einer Temperatur durchgeführt wird, welche oberhalb von Tg des Weichpolymers ist.

15. Verfahren nach Anspruch 10, bei dem ein passendes Flockungsmittel zur Unterstützung der Ausflokkung verwendet wird.

16. Verfahren nach Anspruch 15, bei dem das Flockungsmittel direkt auf den Latex wirkt.

17. Verfahren nach Anspruch 16, bei dem das passende Flockungsmittel ein Kation wie beispielsweise $Na^+$, $Mg^{2+}$, $Ba^{2+}$, $Al^{3+}$ oder ein Kation von Uranoxid ist, oder von diesen Kationen abgeleitet ist.

18. Verfahren zur Herstellung eines Produkts aus einer Enzymreaktion, wobei das Verfahren den Schritt zum in-Kontakt-Bringen eines Substrats mit einer Zusammensetzung nach Anspruch 1 unter Bedingungen umfaßt, so daß das Enzym auf das Substrat reagiert.

19. Verfahren nach Anspruch 18, bei dem die Zusammensetzung Zellen nach Anspruch 6 umfaßt.

**Revendications**

1. Composition dérivée d'un mélange étroit d'un latex aqueux d'un polymère formant un film ou un revêtement et de cellules d'un organisme.

2. Composition suivant la revendication 1, sous la forme d'une pâte ou d'un mastic.

3. Composition suivant la revendication 1, sous la forme d'une masse façonnée, comme des fibres ou des granulés.

4. Composition suivant la revendication 1, dans laquelle la Tv du polymère est inférieure à la plus haute température à laquelle peut résister l'enzyme dans la cellule sans en être exagérément affectée.

5. Composition suivant l'une quelconque des revendications précédentes, dans laquelle les cellules comprennent des cellules bactériennes.

6. Composition suivant l'une quelconque des revendications précédentes, dans laquelle les cellules bactériennes comprennent Pseudomonas sp., Arthrobacter sp., Bacillus sp. ou Enterobacteriacae sp.

**7.** Composition suivant la revendication 1, dans laquelle les cellules comprennent au moins l'une des enzymes suivantes : glucose isomérase, déshalogénase, catalase, oxygénase ou cyanure hydratase.

**8.** Composition suivant l'une quelconque des revendications précédentes, dans laquelle le polymère est un copolymère de méthacrylate de méthyle, un copolymère de styrène, un poly(chlorure de vinylidène) et des copolymères convenables à ce propos ou des copolymères de butadiène-styrène.

**9.** Composition suivant l'une quelconque des revendications précédentes, caractérisé de plus en ce que le solide particulaire comprend à la fois un polymère dur et un polymère mou.

**10.** Procédé pour la préparation d'une composition pour fournir une enzyme utilisée dans un procédé chimique ou biochimique, laquelle composition comprend un agglomérat de cellules d'un organisme comprenant une enzyme étroitement dispersée dans une structure qui est ou dérive d'un ensemble de plusieurs particules d'un solide organique, caractérisé (i) en ce que le solide particulaire comprend au moins un polymère mou qui a une fluidité suffisante pour s'écouler ou qui subit au moins une coalescence partielle de telle sorte qu'une partie au moins des cellules de l'organisme est revêtue de ce polymère, dans lequel cette fluidité suffisante ci-dessus est obtenue à une température qui est inférieure à la température la plus élevée à laquelle peut résister l'enzyme à l'intérieur de la cellule immobilisée intégrale particulière, et (ii) en ce que le procédé comprend au moins les étapes de :

(A) mélange des cellules de l'organisme avec un latex d'au moins un polymère mou, lequel polymère possède suffisamment de fluidité pour pouvoir s'écouler ou subir au moins une coalescence partielle pour recouvrir au moins une partie des cellules de l'organisme de façon à former un mélange étroit ;

(B) des agglomérats sont laissés se former dans le mélange étroit ou leur formation est provoquée ; et

(C) récupération des agglomérats, dans lequel dans l'une au moins des étapes A, B ou C, les particules de polymère s'écoulent ou subissent au moins une coalescence partielle pour recouvrir au moins une partie des cellules.

**11.** Procédé suivant la revendication 10, dans lequel pendant au moins une partie du temps pris par l'étape B ou après celle-ci, la température est maintenue au-delà de la Tv du polymère mou.

**12.** Procédé pour la préparation d'une composition suivant l'une quelconque des revendications 1 à 9, lequel procédé comprend des étapes de :

(A) formation d'un latex aqueux d'au moins un polymère filmogène ;

(B) mélange de ce latex aqueux avec des cellules d'un organisme ; et

(C) des agglomérats sont laissés se former dans le mélange étroit ou leur formation est provoquée.

**13.** Procédé suivant les revendications 10, 11 ou 12, caractérisé de plus en ce que la composition est soumise à un traitement de façonnage.

**14.** Procédé suivant les revendications 10, 11 ou 12, dans lequel une étape au moins du procédé est conduite à une température qui est supérieure à la Tv du polymère mou.

**15.** Procédé suivant la revendication 10, dans lequel un floculant convenable est utilisé pour favoriser la floculation.

**16.** Procédé suivant la revendication 15, dans lequel le floculant agit directement sur le latex.

**17.** Procédé suivant la revendication 16, dans lequel le floculant convenable est ou dérive d'un cation, tel que $Na^+$, $Mg^{2+}$, $Ba^{2+}$, $Al^{3+}$ ou un cation d'oxyde d'uranium.

**18.** Procédé pour la production d'un produit d'une réaction enzymatique, lequel procédé comprend l'étape de mise en contact d'un substrat avec une composition suivant la revendication 1 dans des conditions telles que l'enzyme réagisse sur le substrat.

**19.** Procédé suivant la revendication 18, dans lequel la composition comprend des cellules telles que définies dans la revendication 6.